# EUROPEAN PATENT SPECIFICATION

(11) **EP 1 191 037 B1**
(45) Date of publication and mention of the grant of the patent: **21.09.2005**
(21) Application number: 00942469.8
(22) Date of filing: 03.07.2000
(51) Int. Cl.: C07K 14/805, G01N 33/72, A61K 38/42

(54) **MEANS OF STABILIZING HEMOGLOBINS**
MITTEL ZUR STABILISIERUNG VON HÄMOGLOBINEN
MOYENS POUR STABILISER LES HEMOGLOBINES

(30) Priority: 01.07.1999 JP 18813299
(43) Date of publication of application: 27.03.2002
(73) Proprietor: INTERNATIONAL REAGENTS CORPORATION, Hyogo 651-0083 (JP)
(72) Inventor: KAKUYAMA, Tsutomu, International Reagents Corp., Kobe-shi, Hyogo 651-22 (JP)
(74) Representative: Forstmeyer, Dietmar
(86) International application number: PCT/JP2000/004440
(87) International publication number: WO 2001/002438

(56) References cited:
- EP-A- 0 170 247
- WO-A-91/09615
- WO-A-96/34889
- WO-A-99/18979
- WO-A1-90/11527
- JP-A- 8 245 421
- US-A- 5 051 353

## Description

### Field of the Invention

The present invention relates to a method for stabilizing hemoglobin and a composition containing the same.

### Background of the Invention

Hemoglobin is a hem-protein existed in red blood cell, which is constituted with tetramer consisting of 2 pair of polypeptide chains, (each chain is bound to 1 mol hem), named as α- and β-chains, and having 65,000 of molecular weight. The hemoglobin are contained in blood in an amount of 16 to 18 g/dL for male, and 14 to 16 g/dL for female, and play an oxygen transporting function by reversively detaching oxygen to iron molecule.

Because of maintaining such a rule, a measurement of the hemoglobin is one of the basic measurement items in a clinical laboratory test, and is utilized as a diagnosis of anemia, i.e., iron deficiency anemia, hypoplastic anemia, hemolytic anemia, and etc., in combination with red corpuscle number and hematocric value. Recently, by detecting an extremely small amount of hemoglobin contained in a stool, the measurement takes advantage in determination for a diagnosis of colon cancer.

On the other hand, a glycated hemoglobin is produced by non-enzyme reaction between hemoglobin and glucose. A measurement of the glycated hemoglobin in blood is reflected with a control of blood sugar concentration for past 3 or 4 weeks, and is effectively available as an indication of blood sugar concentration in long term, which is not affected by daily meals. In the present specification, hereinafter, a term of hemoglobin is used, as totally including hemoglobin and glycated hemoglobin.

At present, the measurement of the hemoglobin in clinical laboratory takes place with HPLC method, immunological method, affinitymethod, electrophoresis method, isoelectric fraction method, TBA (2-thiobarbituric acid) method, RIA (Radio Immuno Assay) method, Photic acid method, furosine determination method, and the like.

On measuring the hemoglogin above, a substance which may be of standard and control material, is required. However, the hemoglogins are unstable, and lose oxygen binding function, and a hem iron is changed from Fe²⁺ to Fe³⁺, by oxidation and are changed in color from clear red to dark brown. Furthermore, the hemoglobin are noted to be oxidized to methemoglobin, even by exposing thereof to air, and by lyophilization.

As a method for protecting such an oxidization, there is a method for which, on a site, where an oxygen is to be bound, two moles atoms, such as CO, NO, CN, etc., which have almost the same diameter as of oxygen, are previously bound, or a method for adding sodium azide on lyophilization step. However, these methods have drawbacks that operations are in troublesome; one of the methods provides significantly inadequate low amount, or another is impossible operability. Besides these, a disposal trouble on sodium azide arose due to poisoness. Further, even though a method for adding a compound containing nitrogen is disclosed in the publication (Laid-open Japanese Patent Publication No. Sho 60-35270), an effect for preventing separation of free iron form from hemoglobin is merely disclosed. A method for incorporating glucose and amino acids (Laid-open Japanese Patent Publication No. Sho 61-1620) discloses a protecting agent for oxidizing hemoglobin to methemoglobin, an obj ect of the method, however, is to stabilize hemoglobin which are used as a substitute blood having a concentration mg/mL level, as hemoglobin. Thus, these methods are not acceptable for stabilizing hemoglobin having a concentration in ng to µg/mL, which is required in clinical laboratory test as a standard or control material. Another method for stabilizing hemoglobin by incorporating amino acid and albumin is published (Laid-open Japanese Patent Publication No. Hei 8-245421), however, the method is restricted in a composition of the agent, because this method is required to use albumin other than amino acid.

As discussed above, conventional method for stabilizing the hemoglobin is not suitable as a method for stabilizing control material and standard material, which are required for accurate measuring hemoglobin in a clinical laboratory test. Accordingly, the method for stabilizing control material and standard material containing hemoglobin, are highly desired in the field of pharmaceutical and clinical laboratory test.

### Description of the Invention

As the result of a study on a stabilization of the control material and the standard material, the present inventors were found that the stability of hemoglobin were improved by incorporating with sulfur containing material, particularly compound with SH group, wherein said sulfur containing compound is added in an amount of 0.01 to 0.00001 parts by weight per 1 part by weight of the hemoglobin thereby establishing the present invention.

That is, one of the gists of the present invention is to provide a stabilizing agent for hemoglobin, characterized by stabilizing hemoglobin in a state of solution.

The sulfur containing agent of the present invention is of compound with SH group.

Besides these, according to the present invention, said compound with SH group may be one of the compound selected from the group consisting of, sulfur containing amino acid, such as cysteine, methionine, cystine, and etc., and family thereof; and sulfur containing compound, such as thioglycol acid, 1-thioglycelin, thiodiglycol, mercaptoethanol, glutathione, dithiothreitol and etc., and family thereof.

According to the present invention, compound with SH group may be cysteine and family thereof.

According to the present invention, said hemoglobin may be hemoglobin.

According to the present invention, said hemoglobin may be glycated hemoglobin.

Another gist of the present invention is a method for stabilizing of the present invention.

Further gist of the invention is a composition characterized by incorporating with the stabilizing agent for hemoglobin of the present invention.

Still further gist of the present invention is at least incorporating the stabilizing agent for hemoglobin and hemoglobin of the present invention.

Still another gist of the invention is use of the sulfur containing compound in the stabilizing agent of the present invention.

### Best Mode for carrying out the Present Invention

According to the present invention, the hemoglobin mean hemoglobin and glycated hemoglobin. An example of the glycated hemoglobin includes HbA_{1c}. And another example of the hemoglobin includes HbA₁ₐ, HbA_{1b}, HbF, HbA₀, HbA₂, oxyhemoglobin, carbonylhemoglobin, alkali modified hemoglobin, and the like, and other heterohemoglobin. Besides these, modified hemoglobin, such as phosphate ester derivatives of hemoglobin, hemoglobin-polyalkylene conjugates, hemoglobin-inuren conjugates, and hemoglobin-haptglobin complexes, are also included in the present invention. Further, the hemoglobin of the present invention may be available not only in human origin, but also animal origin, for example, cow, pig, sheep, horse, dog, monkey, rabbit, chicken, and the like. These are used as a standard or control material for various clinic laboratory test. A product offered to these purposes is preferably supplied in a dried substance, as a lyophilized agent, and maybe in a form of dried agent, liquid agent, etc., if desired.

The first and principal gist of a stabilizing method and stabilizing agent for hemoglobin according to the present invention, resides in insuring a stability of the hemoglobin in a liquid state. It is meant by securing stability of the hemoglobin in dried remedy and in a state of solution.

A stabilizing method for the hemoglobin according to the present invention is to incorporate sulfur containing compound to stabilize thereof. Addition of the sulfur containing compound is more convenient to add in a process for preparing agent, however, may be attained by conventionally adding after dissolving the agent, on demand. The hemoglobin standard material and control material, in which the hemoglobin is the main ingredient and the sulfur containing compound is contained as a stabilizing agent, are provided, on adding the sulfur containing agent in a step for preparing agent. If the agent is to be the type, of which the compound is added on demand, the main ingredient and the stabilizing agent containing sulfur containing agent are separately prepared.

As the sulfur containing agent according to the present invention, compound with SH group is well-known and widely available. Compound with SH group includes sulfur containing amino acid, such as cysteine, methyonin, etc.; sulfur containing compound, such as thiobenzoic acid, thioglycol acid, 1-thioglycerine, thiodiglycol, mercaptoethanol glutathione, dithiothreitol, etc., and family thereof. These may be used singly or in combination. The most preferable is the sulfur containing amino acid, such as cysteine, methionine, cystine, and the like, and family thereof. The particularly preferably is cysteine and family thereof.

Amount of the sulfur containing agent to be used is 0.01 to 0.00001 part by weight per 1 part by weight of the hemoglobin, preferably 0.001 to 0.0001 part by weight. A concentration of a solution, when the agent is prepared in a form of solution, is 0.01 to 100 mM, preferably 0.1 to 10 mM, per hemoglobin of 2 to 10 weight % concentration.

The standard material or control material containing hemoglobin, which is provided and assures stability according to the present invention, may be said as a composition containing hemoglobin. Said composition may be incorporated under applying a well-known techniques, in addition of the main ingredient and stabilizer, as base component, with bulking agents, pH control agents for protecting turbidity on solubility thereof, protein, sucrose, high molecular weight compounds, inorganic salts, chelating agents, and the like, or may be combined thereof, on demand.

Example thereof includes various buffer solution or pH control agent, which is capable of controlling pH of 5 to 9, protein such as albumin, gelatin, etc., saccharide, such as glycerol, sucrose, (preferably disaccharide such as sucrose), etc., polysaccharide, such as sodium sulfate dextran, heparin, sodium sulfate, chondroitin, dextran, etc., and high molecular compound such as polyethyleneglycol, polyvinylalcohol, polyvinyl pyrroridone, polypropylene glycohol, etc. These materials may be used singly, or in combination. Besides these, saccharides such as glucose, maltose, inositol, fructose, glucitol, glucono-δ-lactone, trehalose, maltitol, raffinose, mannitol; inorganic compound such as sodium chloride, sodium phosphate, potassium chloride, calcium lactate, etc., chelating agent such as EDTA (ethylenediaminetetraacetic acid), NTA (nitrilotriacetic acid), EDDA (ethylenediaminediacetic acid), CyDTA (trans-1,2-Diaminocycloheane-N,N,N',N'-tetraacetic acid monohydrate), DPTA-OH (1,3-Diamino-2-hydroxyoropane N,N,N',N'-tetraacetic acid), DTPA (Diethylenetriamine-N,N,N',N",N"-pentaacetic acid), EDDP (Ethylenediamine-N,N'-dipropionic acid, dihydrochloride), EDDPO [Ethylenediamine-N, N'-bis(methylenephosphosphonic acid), hemihydrate], EGTA [Ethyleneglycol-bis-(β-amino-ethylether) tetraacetic acid], HBED [N,N'-bis(2-hydroxybenzyl) ethylenediamine-N,N-diacetic acid], HDTA (1,6-Hexamethylenediamine-N,N,N',N'-tetraacetic acid), HIDA [N-(2-Hydroxyethyl) iminodiacetic acid], IDA (Iminodiacetic acid), NTP (Nitrilotripropionic acid), NTPO [Nitrilotris (methylenephosphonic acid), trisodium salt], TTHA (Triethylenetetramine-N,N,N',N",N''',N'''-hexaacetic acid), etc., α-, β-, γ-CD (cyclodextrin), or these CD modified with polymer, and the like. These may be used singly or in combination.

### Examples

The present invention is explained in more detailed with referring to the following examples, which are not extruded as restricting the present invention.

### (Example 1)

The mixture of, an agents stated below, were prepared with or without adding 1 mM L-cysteine, 1 mL mixture were pipetted into 5 mL glass vessel, and were lyophilized to form lyophilized preparation. As hemoglobin, hemoglobin standard containing HbA₁ₐ, HbA_{1b}, HbF, and HbA₀ were used, and added in a concentration of 7% (w/v).

| | |
|---|---|
| 10 mM | Phosphate buffer solution (pH 7.0) |
| 25 mM | EDTA·2Na |
| 7 % | Hemoglobin standard (JML company) |
| 25 % | Sucrose |

### (Experimental Example 1)

Each of the lyophilized agents, with or without incorporating L-cysteine, which were prepared in the Example 1, was dissolved in 1 mL distilled water, to compare stability of the hemoglobin in a state of solution, at 25 °C, after 25 hours elapse of time. Measurement of the hemoglobin was performed by detecting absorbance (O. D. value) at 577 nm. (Method in Enzymology, 188, 266-272). From the results, as shown in the Table 1, by adding L-cysteine, a sufficient stabilizing effect of the hemoglobin in a state of solution was confirmed. Further, a chemical analysis of each portion using an automatic glycated hemoglobin analyzer HLC-723GHbIII, manufactured by Toso company. These results showed that was stable in all kinds of hemoglobin fractions.

**Table 1**

| | 0 Hour | 25 Hour |
|---|---|---|
| With L-cysteine adding | 0.86 | 0.85 |
| Without L-cysteine | 0.86 | 0.41 |

### (Example 2)

A sample was prepared with the agent, stated below, by adding with or without adding 1 mM L-cyteine. In the same manner, as stated in the Example 1, the hemoglobin standard containing HbA_{1c}, HbA₁ₐ, HbF, and HbA₀ was used in order to make a concentration of hemoglobin in 7% (w/v).

| | |
|---|---|
| 10 mM | Phosphate buffer solution (pH 7.0) |
| 25 mM | EDTA 2 Na |
| 7 % | Hemoglobin standard (JML company) |

### (Experimental Example 2)

Each of the agents, with or without incorporating L-cysteine, which were prepared in the Example 2, was studied in comparing stability of hemoglobin after preparation, and at 25 °C after 25 hours elapse of time, in the same manner as of the Experimental Example 1. The results were shown in Table 2. According to adding cysteine, a sufficient stability of the hemoglobin was obtained, regardless of presence of sucrose. Further, chemical analysis of each portion was carried out using Automatic Analyzer of glycohemoglobin HLC-723GHbIII. These results showed that was stable in all kinds of hemoglobin fractions.

**Table 2**

| | 0 Hour | 25 Hour |
|---|---|---|
| With L-cysteine adding | 0.85 | 0.85 |
| Without L-cysteine | 0.85 | 0.43 |

### Possibility in commercially available

By the stabilizing method for hemoglobin and stabilizing agent, characterized in adding sulfur containing compound according to the present invention, the stability of hemoglobin and glycated hemoglobin in a state of solution was secured. Thus, by introducing stabilizing means for hemoglobin, for example, a stability of a standard material and controlling material containing hemoglobin for clinical laboratory test, was improved and was resulted in expecting high accurate laboratory test results. The present invention is useful for contributing in clinical laboratory test and pharmaceutical field.

## Claims

1. An agent for clinical laboratory tests **characterized in** comprising hemoglobin, a sulfur containing compound, and a chelating agent, wherein said sulfur containing compound is added in an amount of 0.01 to 0.00001 parts by weight per 1 part by weight of the hemoglobin.

2. The agent according to claim 1, **characterized by** stabilizing the hemoglobin in a state of solution.

3. The agent according to claim 1 or 2, wherein said sulfur containing compound is a compound with a SH group.

4. The agent according to claim 3, wherein said compound with a SH group is at least one selected from the group consisting of cysteine, methionine, cystine, thiobenzoic acid, thioglycolic acid, 1-thioglycerin, thiodiglycol, mercaptoethanol, glutathione, and thioglycerol.

5. The agent according to claim 3, wherein said compound with a SH group is a sulfur containing amino acid.

6. The agent according to claim 5, wherein said sulfur containing amino acid is cysteine.

7. The agent according to claims 1 to 6, wherein said hemoglobin is glycated hemoglobin.

8. The agent according to claims 1 to 7, **characterized in that** it is a lyophilized agent.

9. The agent according to claims 1 to 7, **characterized in that** it is a liquid agent.

## Patentansprüche

1. Mittel für einen klinischen Labortest, **dadurch gekennzeichnet, dass** es Hämoglobin, eine Schwefel enthaltende Verbindung und ein Chelatisierungsmittel umfasst, wobei die Schwefel enthaltende Verbindung in einer Menge von 0,01 bis 0,000.01 Gewichtsteilen pro 1 Gewichtsteil des Hämoglobins zugegeben wird.

2. Mittel nach Anspruch 1, **dadurch gekennzeichnet, dass** das Hämoglobin im gelösten Zustand stabilisiert wird.

3. Mittel nach Anspruch 1 oder 2, wobei die Schwefel enthaltende Verbindung eine Verbindung mit einer SH-Gruppe ist.

4. Mittel nach Anspruch 3, wobei diese Verbindung mit einer SH-Gruppe mindestens eine ist, die aus der Gruppe ausgewählt wird, welche aus Cystein, Methionin, Cystin, Thiobenzoesäure, Thioglycolsäure, 1-Thioglycerin, Thiodiglycol, Mercaptoethanol, Glutathion und Thioglycerin besteht.

5. Mittel nach Anspruch 3, wobei die Verbindung mit einer SH-Gruppe eine Schwefel enthaltende Aminosäure ist.

6. Mittel nach Anspruch 5, wobei die Schwefel enthaltende Aminosäure Cystein ist.

7. Mittel nach den Ansprüchen 1 bis 6, wobei das Hämoglobin glycosyliertes Hämoglobin ist.

8. Mittel nach den Ansprüchen 1 bis 7, **dadurch gekennzeichnet, dass** es ein lyophilisiertes Mittel ist.

9. Mittel nach den Ansprüchen 1 bis 7, **dadurch gekennzeichnet, dass** es ein flüssiges Mittel ist.

## Revendications

1. Agent destiné à des tests cliniques de laboratoire **caractérisé en ce qu'**il comprend de l'hémoglobine, un composé contenant du soufre, et un agent de chélation, dans lequel ledit composé contenant du soufre est ajouté en une quantité de 0,01 à 0,00001 partie en poids pour 1 partie en poids de l'hémoglobine.

2. Agent selon la revendication 1, **caractérisé par** la stabilisation de l'hémoglobine dans un état de solution.

3. Agent selon la revendication 1 ou 2, dans lequel ledit composé contenant du souffre est un composé ayant un groupe SH.

4. Agent selon la revendication 3, dans lequel ledit composé ayant un groupe SH est au moins un composé choisi dans le groupe constitué par la cystéine, la méthionine, la cystine, l'acide thiobenzoïque, l'acide thioglycolique, la 1-thioglycérine, le thiodiglycol, le mercaptoéthanol, la glutathione et le thioglycérol.

5. Agent selon la revendication 3, dans lequel ledit composé ayant un groupe SH est un acide aminé contenant du soufre.

6. Agent selon la revendication 5, dans lequel ledit acide aminé contenant du soufre est la cystéine.

7. Agent selon les revendications 1 à 6, dans lequel ladite hémoglobine est de l'hémoglobine glyquée.

8. Agent selon les revendications 1 à 7, **caractérisé en ce qu'**il est un agent lyophilisé.

9. Agent selon les revendications 1 à 7, **caractérisé en ce qu'**il est un agent liquide.
